(19) Europäisches Patentamt
European Patent Office
Office européen des brevets

(11) Veröffentlichungsnummer: **0 291 067 B1**

# (12) EUROPÄISCHE PATENTSCHRIFT

(45) Veröffentlichungstag der Patentschrift: **07.09.94**

(51) Int. Cl.5: **C08B 37/16**, C12P 19/18

(21) Anmeldenummer: **88107694.7**

(22) Anmeldetag: **13.05.88**

Die Akte enthält technische Angaben, die nach dem Eingang der Anmeldung eingereicht wurden und die nicht in dieser Patentschrift enthalten sind.

## (54) Verfahren zur Herstellung von Cyclooctaamylose.

(30) Priorität: **14.05.87 DE 3716181**

(43) Veröffentlichungstag der Anmeldung:
**17.11.88 Patentblatt 88/46**

(45) Bekanntmachung des Hinweises auf die Patenterteilung:
**07.09.94 Patentblatt 94/36**

(84) Benannte Vertragsstaaten:
**AT BE CH DE FR GB IT LI NL SE**

(56) Entgegenhaltungen:
**EP-A- 0 220 719**

**ANGEWANDTE CHEMIE, INTERNATIONAL EDITION IN ENGLISH, Band 18, Nr. 8, 1979,Seiten 623-624, Verlag Chemie GmbH, Weinheim, DE; F. VÖGTLE et al.: "Complexesof gamma-cyclodextrin with crown ethers, cryptands, coronates, and cryptates"**

**PATENT ABSTRACTS OF JAPAN, Band 10, Nr. 98 (C-339)[2155], 15. April 1986;& JP-A-60 227 693**

(73) Patentinhaber: **Consortium für elektrochemische Industrie GmbH**
**Zielstattstrasse 20**
**D-81379 München (DE)**

(72) Erfinder: **Schmid, Gerhard, Dr. Dipl.-Biologe**
**Frankplatz 18**
**D-8000 München 45 (DE)**
Erfinder: **Eberle, Hans-Jürgen, Dr. Dipl.-Chem.**
**Ambacherstrasse 36**
**D-8000 München 71 (DE)**

**EP 0 291 067 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Cyclooctaamylose durch enzymatische Spaltung von Stärke in Gegenwart eines Komplexbildners.

Cyclooctaamylose ist relativ gut wasserlöslich, besitzt einen hydrophoben Torus von $10 \cdot 10^{-10}$m Durchmesser, in den Gastmoleküle eingeschlossen werden können. Aufgrund dieser Eigenschaften ist Cyclooctaamylose ein begehrter Einsatzstoff u.a. auf dem Arzneimittelsektor, auf den Gebieten des Pflanzenschutzes, der Kosmetik oder in der Nahrungsmittelindustrie.

Gemäß DE 3317064 A1 ist es Stand der Technik Cyclooctaamylose aus Stärkehydrolysaten durch Fällung mit Brombenzol abzutrennen. Nachteiligerweise ist der Komplexbildner Brombenzol nicht selektiv für Cyclooctaamylose, sondern es wird auch Cycloheptaamylose mitgefällt, welche nur durch aufwendige Methoden abgetrennt werden kann.

In der DE 3446080 A1 bzw. der entsprechenden GB 2151647 A werden Als Komplexbildner für Cyclooctaamylose Phenol- und Benzolderivate genannt, denen gleichzeitig Komplexbildner für Cyclohexaamylose zugegeben werden. Auch hier ist eine Auftrennung von Cycloocta-, Cyclohepta- und Cyclohexaamylose notwendig. Chemical Abstracts 104:128250q, worin die japanische Offenlegungsschrift JP 60,227,693 referiert wird, beschreibt tetra- oder pentacyclische Triterpenoide als Komplexbildner bei der Herstellung von Cyclooctaamylose.

Aufgabe der Erfindung war es ein Verfahren zur Herstellung von Cyclooctaamylose hoher Reinheit zu entwickeln, das verbesserte Ausbeuten liefert und gleichzeitig die Isolierung der Cyclooctaamylose vereinfacht.

Gegenstand der Erfindung ist ein Verfahren zur Herstellung von Cyclooctaamylose durch enzymatische Spaltung einer wässerigen Zubereitung von Stärke in Gegenwart eines Komplexbildners, das dadurch gekennzeichnet ist, daß als selektiver Kompexbildner für Cyclooctaamylose Verbindungen der Formel I

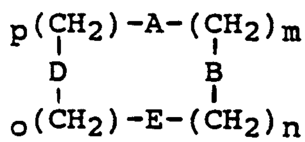

wobei A,B,D und E unabhängig voneinander für

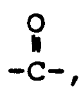, 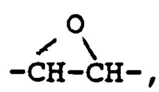,

-O-, -NH-,

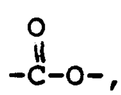,

$-CR_2-$,

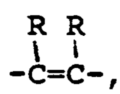, 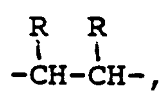,

-C≡C-,

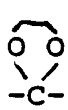 und 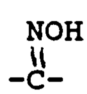

(R = Wasserstoff-, Methyl-, Ethyl-, Hydroxyl-, Methoxy-, Ethoxy-, oder Carboxyrest)
stehen und m, n, o und p innerhalb der Grenzen von 0 bis 20 liegen mit der Maßgabe, daß die Anzahl der Atome die den Ring bilden, innerhalb der Grenzen von 13 bis 24 liegt, eingesetzt werden.

Insbesondere wegen der leichten Zugänglichkeit sind der Wasserstoff- und Methylrest bevorzugt.

Beispiele für Verbindungen der Formel I sind:

a) Makrocyclische ungesättigte Kohlenwasserstoffe wie
Cyclotetradeca-1.8-dien, Cyclopentadeca-1.8-dien, Cyclohexadeca-1.9-dien, Cyclohexa-1.5.9.13-tetraen, 1.5.9.13-Tetramethylcyclohexadeca-1.5.9.13-tetraen, Cyclotetracosa-1.9.17-trien, Cyclohexadeca-1.9-diin und deren Mono- bzw. Polyepoxide wie
15-Oxa-bicyclo[12.1.0]-pentadec-7-en, 8.16-Dioxa-tricyclo[13.1.0.$0^{7.9}$]-hexadecan, 17-Oxa-bicyclo[14.1.0]-heptadec-8-en, 9.18-Dioxa-tricyclo[15.1.0.$0^{8.10}$]-octadecan, 17-Oxa-bicyclo[14.1.0]-heptadeca-4.8.12-trien.

b) Makrocyclische Ketone bzw. Polyketone und Acetale wie
Cyclotridecanon, Cyclotetradec-7-en-1-on, Cycloheptadec-9-en-1-on, Cyclohexadec-8-in-1-on, Cyclotetracosa-1.8.16-trion, Cyclotetracosa-1.8.17-trion, 2-Methylcyclotridecan-1-on, 3-Methylcyclopentadecan-1-on, 17.20-Dioxaspiro[15.4]-cosan, 1.1-Diethoxy-cyclohexadec-8-en.

c) Makrocyclische Alkohole wie
Cyclotetradec-7-en-1-ol, Cyclohexadecan-1.8-diol, Cyclohexadecan-1.9-diol, Cyclohexadeca-5.9.13-trien-1-ol

d) Makrocyclische Alkoxy- bzw. Carboxyverbindungen wie
1-Acetoxy-cyclohexadec-8-en, 1-Methoxy-cyclohexadec-8-en, 1.2-Dimethoxy-cyclohexadec-8-en

e) Makrocyclische Oxa-oxo-Verbindungen wie
2.5-Dioxa-1.6-dioxo-cyclotetradecan, 2.5-Dioxa-1.6-dioxo-cyclohexadecan, 2.5-Dioxa-1.6-dioxo-cycloheptadecan, 2.5-Dioxa-1.6-dioxo-cycloeicosan, 3-Methyl-2.5-dioxa-1.6-dioxo-cyclohexadecan, 2.8-Dioxa-1-oxo-cycloheptadecan, 2.7-Dioxa-1-oxo-cycloheptadecan.

f) Makrocyclische Hydroxyiminoverbindungen wie
2-Oxa-1-oxo-6-hydroxyimino-cyclohexadecan, 1-Hydroxyiminocyclotridecan,1-Hydroxyiminocyclohexadec-8-en

g) Makrocyclische Mono- bzw. Oligo-Azaverbindungen wie
2-Aza-1-oxo-cyclotridecan, 2.8-Diaza-1.9-dioxo-cyclohexadecan, 2-Aza-1-oxo-cyclotetradec-7-en

Vorzugsweise werden Verbindungen der Formel I, bei denen die Anzahl der Atome, die den Ring bilden, innerhAlb der Grenzen von 13 bis 18 liegt, verwendet. Insbesondere sind dies Cyclotridecanon, Cyclotetradecanon, Cyclotetradec-7-en-1-on, 15-Oxabicyclo[12.1.0]-pentadec-7-en, 8.16-Dioxa-tricyclo[13.1.0.$0^{7.9}$]-hexadecan, Cyclotetradeca-1.8-dion, Cyclopentadec-8-en-1-on, Cyclopentadeca-1.8-dion, 16-Oxa-bicyclo-[13.1.0]-hexadec-7-en, 8.17-Dioxa-tricyclo[14.1.0.$0^{7.9}$]-heptadecan, Cyclohexadec-8-en-1-on, Cyclohexadeca-1.9-dion, Cyclohexadeca-1.8-dion, Cyclohexadeca-1.9-dien, 17-Oxabicyclo[14.1.0]-cycloheptadec-8-en, 9.18-Dioxa-tricyclo-[15.1.0.$0^{8.10}$]-cyclooctadecan, Cycloheptadec-9-en-1-on, Cyclohepta-1.9-dion, 18-Oxa-bicyclo-[15.1.0]-cyclooctadec-8-en, 2.5-Dioxa-1.6-dioxo-cyclohexadecan, 2.5-Dioxa-1.6-dioxo-cycloheptadecan, 2.8-Dioxa-1-oxo-cycloheptadecan, 1.7-Dioxa-1-oxo-cycloheptadecan, 2-Oxa-1-oxo-cyclotetradecan, 2-Oxa-1-oxo-cycloheptadecan, 2-Oxa-1-oxo-cyclopentadecan.

Mann kann nativer Stärke oder Maltodextrine mit einem Dextroseäquivalent <15 einsetzen.

Als wässerige Zubereitungen von Stärke können 4 bis 40 Gew.-%-ige wässerige Lösungen von gelifizierter Stärke eingesetztwerden. Sie werden im einfachsten Fall durch Kochen entsprechender Mengen von Stärke in Wasser gewonnen.

Die genannten Zubereitungen enthAlten zur Enzymstabilisierung zumeist kleinere Mengen an Calciumchlorid, insbesondere 5-10mMol/l.

Den genannten Stärkezubereitungen wird nun das an sich bekannte Enzym Cyclodextringlycosyltransferase zugegeben. Als Quelle für dieses Enzym dienen Mikroorganismen wie Bacillus macerans (Zentr. Bakteriol, Parasitenk., Abt. II, 14, 722 (1905)), Bacillus stearothermophilus (US 3988206), Bacillus subtilis No. 313 (Agric. Biol. Chem 50, 8, 2161-2162 (1986)), Bacillus circulans (US 4477568), Bacillus ohbensis (JP 52-31949), Bacillus megaterium (US 3812011), Bacillus spec. No. 17-1 (US 3923598),Klebsiella pneumoniae M5 a L (Arch. Microbiol. 111, 271 (1977)), Micrococcus luteus oder Micrococcus varians (beide EP 0017242).

Das Enzym wird vorzugsweise in solchen Mengen zugegeben, daß das Gewichtsverhältnis von Enzym : Stärke 1:2000 bis 1:50000, insbesondere 1:5000 bis 1:20000 beträgt.

Die Menge des Komplexbildner sind 1-20 Gew.-% bezogen auf das Gewicht der eingesetzten Stärke, insbesondere 8-15 Gew.-%, eingesetzt.

Vorzugsweise wird beim erfindungsgemäßen Verfahren sofort nach der Zugabe des Enzyms der Komplexbildner eingesetzt.

Der pH-Wert der Stärkebereitung beträgt 4,0 bis 11,0, insbesondere 6,0 bis 9,5.

Die Spaltungsreaktion wird vorzugsweise bei Temperaturen von 30-60 °C, insbesondere 40-50 °C unter Rühren durchgeführt, wobei die Reaktionszeit vorzugsweise 10-48 Stunden beträgt.

Die Reaktionskontrolle kann beispielsweise durch Probenentnahme und chromatographische Analyse (HPLC-Methode, Agric. Biol. Chem. 49, 4, 1189-1191 (1985)) erfolgen.

Zur Aufbereitung wird der unlösliche Cyclooctaamylosekomplex, durch bekannte Methoden z.B. Dekantieren, Filtrieren, Zentrifugieren von den übrigen Reaktionspartnern abgetrennt. Nachfolgend wird der Cyclooctaamylosekomplex wieder in Cyclooctaamylose und Komplexbildner getrennt. Eine geeignete Methode hierfür ist die Behandlung mit heißem Wasser oder Wasserdampf, wobei nach Art einer Wasserdampfdestillation der Komplexbildner aus dem Gemisch entfernt wird. Eine weitere Methode besteht in der Extraktion des wasserhaltigen Cyclooctaamylosekomplexes mit einem organischen Lösungsmittel. Beispiele für solche Lösungsmittel sind Toluol oder Cyclohexan.

Man erhält bis zu 48Gew.-% Cyclooctaamylose (bezogen auf eingesetze Stärke) mit einer Reinheit bis zu 95%.

Diese Cyclooctaamylose wird vorzugsweise in an sich bekannter Weise mit Glucoamylase zur Entfernung von verschleppter Reststärke behandelt und nachfolgend vorzugsweise mit einem organischen Lösungsmittel wie Alkoholen z.B. Isopropanol, Methanol oder Aceton versetzt, worauf nach längerem Stehen Cyclooctaamylose mit einer Reinheit von 95->99,9% auskristallisiert.

Cyclooctaamylose, hergestellt nach dem erfindungsgemäßen Verfahren, findet unter anderem Verwendung als Bestandteil von Pflanzenschutzmitteln, Medikamenten, Kosmetika oder Lebensmitteln.

Beispiel 1

20g lösliche Kartoffelstärke wurden in 200ml Wasser suspendiert, das 4 mmol Tris(hydroxymethyl)-aminomethanhydrochlorid (pH 7,2) und 1 mmol Calciumchlorid enthielt.
Durch Erhitzen auf 95 °C für 25min wurde die Stärke gelifiziert. Nach Abkühlen auf 50 °C wurden 2mg Cyclodextringlycosyltransferase von Bacillus macerans und 2,5 g Cyclohexadec-8-en-1-on zugegeben. Unter kräftigem Rühren wurde der Ansatz für 36h bei 50 °C inkubiert. Durch HPLC-Analyse eines Aliquots der Reaktionsmischung stellte sich heraus, daß 46Gew.-% der eingesetzten Stärke zu Cyclooctaamylose umgesetzt wurden.

Der schwerlösliche Cyclooctaamylose-Cyclohexadec-8-en-1-on-Komplex wurde durch Zentrifugieren abgetrennt. Durch Aufnehmen in 200ml Wasser und anschließendes Abzentrifugieren wurde der Komplex zweimal gewaschen. Der Komplex wurde daraufhin in 200ml Wasser aufgenommen und das Cyclohexadec-8-en-1-on als Azeotrop mit Wasser abdestilliert. Die resultierende Cycloamyloselösung enthielt Cyclooctaamylose mit einer Reinheit von 92%.
Zur Entfernung der noch vorhandenen Reststärke wurde die Cycloamylosekonzentration auf 40 Gew.-% eingestellt und mit 0,5mg Glucoamylase über Nacht bei 55 °C, pH 5 inkubiert. Danach wurde der Ansatz auf Raumtemperatur abgekühlt, das gleiche Volumen Isopropanol zugegeben und für 4 Stunden bei 4 °C stehen gelassen. Der gebildete Niederschlag wurde abgetrennt, mit Isopropanol gewaschen und im Vakuumtrockenschrank bei 60 °C getrocknet. Die Ausbeute an Cyclooctaamylose betrug 7,6g. Die Reinheit war >99%.

Beispiel 2

Das Beispiel 1 wurde wiederholt, jedoch an Stelle des Enzyms von Bacillus macerans wurden 1mg Cyclodextringlycosyltransferase des Alkalophilen Bacillus Nr. 17-1 (ATCC 30007) verwendet.
Der pH des Reaktionsansatz wurde auf 9 eingestellt. Es wurden 8g reiner Cyclooctaamylose erhalten.

Beispiel 3 (Vergleichsversuch) und Beispiele 4-10

Bei den Beispielen 3-10 der Tabelle wurden folgende standardisierte Reaktionsansätze verwendet:
5g Kartoffelstärke wurden in 50ml Pufferlösung (siehe Beispiel 1) suspendiert und gelifiziert. 0,5mg Cyclodextringlycosyltransferase von Bacillus macerans wurden eingesetzt. Die Reaktionstemperatur war 50 °C. Inkubationszeiten t in Stunden und Rohausbeuten an Cyclooctaamylose (in % bezogen auf eingesetzte Stärke), die mit den jeweiligen Komplexbildnern erreicht wurden, sind in der Tabelle zusammengefaßt.

Tabelle:

| Beispiel | Komplexbildner | t | Rohausbeute |
|---|---|---|---|
| 3 | Cyclododecanon | 48 | 1,5 |
| 4 | Cyclotridecanon | 42 | 42 |
| 5 | Cyclotetradec-7-en-1-on | 42 | 45 |
| 6 | Cyclohexadecan-1.9-dion | 48 | 43 |
| 7 | 9.18-Dioxa-tricyclo-$[15.1.0.0^{8.10}]$-cyclo-octadecan | 48 | 38 |
| 8 | 2.8-Dioxa-1-oxo-cyclo-heptadecan | 48 | 39 |
| 9 | 2-Oxa-1-oxo-cyclohepta-dec-7-en | 48 | 34 |
| 10 | 2.5-Dioxa-1.6-dioxo-cyclolohexadecan | 48 | 38 |

## Patentansprüche

**1.** Verfahren zur Herstellung von Cyclooctaamylose durch Behandlung einer 4 bis 40%igen wässerigen Zubereitung, enthaltend native Stärke oder Maltodextrine mit einem Dextroseäquivalent < 15 mit einem pH-Wert von 4,0 bis 11,0 mit CGTase in Gegenwart eines Komplexbildners in Mengen von 1-20 Gew.-%, bezogen auf das Gewicht der eingesetzten Stärke, und anschließende Abtrennung des unlöslichen Cyclooctamylosekomplexes durch Dekantieren, Filtrieren und/oder Zentrifugieren von den übrigen Reaktionspartnern, dadurch gekennzeichnet, daß als selektiver Kompexbildner für Cyclooctaamylose Verbindungen der Formel I

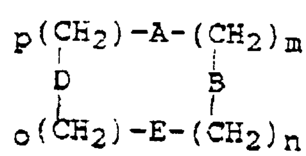

wobei A,B,D und E unabhängig voneinander für

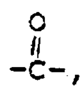, 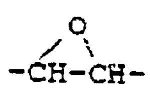,

-O-, -NH-,

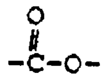,

$-CR_2-$,

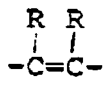, 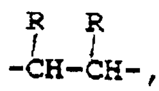,

$-C\equiv C-$,

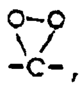, und 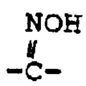

(R = Wasserstoff-, Methyl-, Ethyl-, Hydroxyl-, Methoxy-, Ethoxy- oder Carboxyrest) stehen und m, n, o und p innerhalb der Grenzen von 0 bis 20 liegen mit der Maßgabe, daß die Anzahl der Atome die den Ring bilden, innerhalb der Grenzen von 13 bis 24 liegt, eingesetzt werden.

**Claims**

**1.** Process for preparing cyclooctaamylose by treatment of a 4 to 40% aqueous preparation containing native starch or maltodextrins having a dextrose equivalent < 15 having a pH of from 4.0 to 11.0 with CGTase in the presence of a complexing agent in amounts of 1-20% by weight, based on the weight of the starch employed, and subsequent separation of the insoluble cyclooctaamylose complex from the other reactants by decantation, filtration and/or centrifugation characterized in that, as a selective complexing agent for cyclooctaamylose, compounds of the formula I

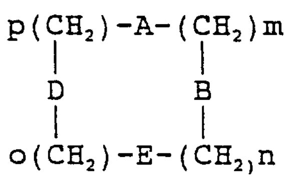

where A, B, D and E, independently of one another, represent

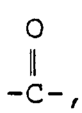, 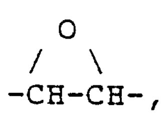,

-O-, -NH-,

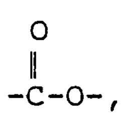,

$-CR_2-$,

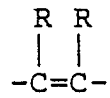, 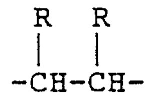,

-C≡C-,

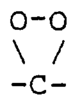 and 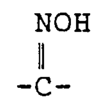

(R = hydrogen, methyl, ethyl, hydroxyl, methoxy, ethoxy or carboxyl radical)
and m, n, o and p are within the limits 0 to 20, with the proviso that the number of atoms forming the ring is within the limits from 13 to 24, are employed.

## Revendications

1. Procédé pour la préparation de la cyclo-octaamylose par traitement d'une préparation aqueuse ayant une teneur de 4 à 40 %, en amidon natif ou en maltodextrines avec un équivalent dextrose < 15, avec une valeur de pH de 4,0 à 11,0 avec de la CGTase en présence d'un agent complexant en quantités de 1 à 20 % en poids, par rapport au poids de l'amidon utilisé, et ensuite par séparation du complexe de cyclo-octa-amylose insoluble par décantation, filtration et/ou centrifugation, des autres partenaires de réaction, caractérisé en ce qu'on utilise en tant qu'agent complexant sélectif pour la cyclo-octa-amylose des composés de formule I

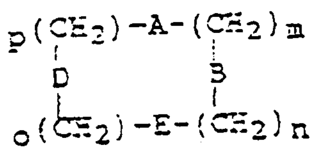

A, B, D et E, indépendamment les uns des autres, représentant les radicaux

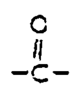, 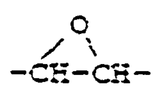,

-O-, -NH-,

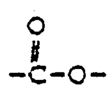,

$-CR_2-$,

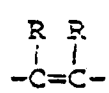, 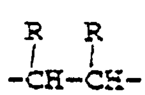,

-C≡C-,

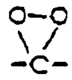, et 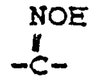

(R = les radicaux hydrogène, méthyle, éthyle, hydroxyle, méthoxy, éthoxy ou carboxy)
et m, n, o et p se trouvant à l'intérieur des limites de 0 à 20, à la condition que le nombre d'atomes qui forment le cycle se trouve à l'intérieur des limites de 13 à 24.